# EUROPEAN PATENT APPLICATION

(11) **EP 1 911 392 A2**
(43) Date of publication of application: **16.04.2008**
(21) Application number: 07253898.6
(22) Date of filing: 02.10.2007
(51) Int. Cl.: A61B 1/12, A61B 1/313

(54) **Apparatus for cleaning a distal scope end of a medical viewing scope**

(30) Priority: 03.10.2006 US 542060
(71) Applicant: Ethicon Endo-Surgery, Inc., Cincinatti, OH 45242 (US)
(72) Inventor: Weisenburgh II, William B., Maineville Ohio 45039 (US); Gill, Robert P., Mason Ohio 45040 (US); Hess, Christopher J., Cincinnati Ohio, 45206 (US); Cummings, John F., Madiera Ohio, 45243 (US)
(74) Representative: Tunstall, Christopher Stephen

(57) **Abstract**

A first apparatus for cleaning a distal end of a medical scope includes an annular sheath surroundingly attachable to the scope wherein the distal scope end is in fluid communication with the distal end of a lumen of the attached sheath with the proximal lumen end fluidly connectable to an irrigation fluid source and/or vacuum source. A second apparatus includes a motor-driven rotatable cannula having a closed distal end. A third apparatus includes an annular sheath and a lens which closes off the sheath, wherein the scope is insertable into the sheath, and wherein the lens is exposed to a distal lumen end of a lumen of the attached sheath. A fourth apparatus includes an annular sheath and a transparent shield rotatable attachable to the sheath to seal the distal sheath end. A fifth apparatus includes a sleeve attachable to and slidable along a scope.

## Description

### Field of the Invention

The present invention is related generally to medical equipment, and more particularly to apparatus for cleaning a distal scope end of a medical viewing scope.

### Background of the Invention

During some conventional laparoscopic procedures, first and second trocars are used to create two openings in the patient's abdomen. A rigid laparoscope is inserted through the first trocar to visualize patient tissue. A treating medical instrument is inserted through the second trocar to treat the patient tissue being visualized with the laparoscope. Bodily fluid dispersion and floating debris have a tendency to accumulate on the scope lens of the inserted laparoscope degrading the clarity of the view. Thus, at times during the laparoscopic procedure the laparoscope must be withdrawn from the first trocar and the scope lens wiped to remove the accumulated material which caused the blurred viewing. The removal of the laparoscope is inconvenient and causes delays in the laparoscopic procedure. Upon reinsertion of the laparoscope, it is necessary for the physician to take additional time to maneuver the scope to reacquire the patient tissue of interest.

### Summary

A first expression of a first embodiment of the invention is for apparatus for cleaning a distal scope end of a medical viewing scope. The apparatus includes an annular sheath having open proximal and distal sheath end portions. The sheath is surroundingly attachable to the scope and is insertable into a patient. The sheath includes a tubular wall having inside and outside diameters and containing a lumen between the inside and outside diameters. The lumen has proximal and distal lumen ends. The proximal lumen end is fluidly connectable to at least one of an irrigation fluid source and a vacuum source. The distal scope end is in fluid communication with the distal lumen end of the attached sheath.

A second expression of a first embodiment of the invention is for apparatus for cleaning a distal scope end of a medical viewing scope. The apparatus includes an annular sheath having open proximal and distal sheath end portions. The sheath is surroundingly attachable to the scope and is insertable into a patient. The sheath includes a tubular wall having inside and outside diameters and containing a lumen between the inside and outside diameters. The lumen has proximal and distal lumen ends. The proximal lumen end is fluidly connectable to at least one of an irrigation fluid source and a vacuum source. The distal scope end is in fluid communication with the distal lumen end of the attached sheath. The distal sheath end portion has an annular manifold which is in fluid communication with the distal lumen end and which includes at least one orifice pointing toward the distal scope end.

A first expression of a second embodiment of the invention is for apparatus for cleaning a distal scope end of a medical viewing scope. The apparatus includes a motor-driven rotatable cannula having an open proximal cannula end and having a transparent and closed distal cannula end. The scope is insertable into the proximal cannula end with the distal scope end disposed proximate and spaced apart from the distal cannula end. Rotation of the cannula helps to expel any material that has accumulated on the distal cannula end when the cannula is inserted into a patient.

A first expression of a third embodiment of the invention is for apparatus for cleaning a distal scope end of a medical viewing scope. The apparatus includes an annular sheath and a lens. The sheath has open proximal and distal sheath ends. The lens is positioned in and attached to the sheath and closes off the sheath proximate the distal sheath end. The lens has a distal surface. The scope is insertable into the proximal sheath end with the distal scope end disposed proximate the lens. The sheath is insertable into a patient. The sheath includes a tubular wall having inside and outside diameters and containing a lumen between the inside and outside diameters. The lumen has proximal and distal lumen ends. The proximal lumen end is fluidly connectable to at least one of an irrigation fluid source and a vacuum source. The distal surface of the lens is in fluid communication with the distal lumen end.

A first expression of a fourth embodiment of the invention is for apparatus for cleaning a distal scope end of a medical viewing scope. The apparatus includes an annular sheath having open proximal and distal sheath ends and a rotatably-driven transparent shield rotatably attachable to the sheath proximate the distal sheath end to seal the distal sheath end. The scope is insertable into the proximal sheath end with the distal scope end disposed proximate and spaced apart from the shield. Rotation of the shield helps to expel any material that has accumulated on the shield when the sheath and the attached shield are inserted into a patient.

A first expression of a fifth embodiment of the invention is for apparatus for cleaning a distal scope end of a medical viewing scope. The apparatus includes a sleeve attachable to and slidable along the scope. The sleeve includes a distal sleeve tip. The distal sleeve tip of the attached sleeve is slidably extendable from and slidably retractable from the distal scope end.

Several benefits and advantages are obtained from one or more of the expressions of embodiments of the invention which provide cleaning of a distal scope end of a medical viewing scope while the scope remains inserted in a patient. In one example, not removing the scope for cleaning and not reinserting the cleaned scope reduces the time for a laparoscopic procedure. In the same or a different example, not removing the scope for cleaning and not reinserting the cleaned scope keeps the inserted scope aligned with the patient tissue of interest during cleaning so that the physician does not have to take additional time to maneuver the scope to reacquire the patient tissue of interest.

### Brief Description of the Figures

FIGURE 1 is a schematic view of a first embodiment of the invention including a handpiece, wherein a sheath is surroundingly attached to a scope and is inserted into a patient using a trocar, and wherein the sheath and the trocar are shown in cross section;

FIGURE 2 is a view of the sheath of Figure 1;

FIGURE 3 is a cross sectional view of the sheath of Figure 2 taken along lines 3-3 of Figure 2;

FIGURE 4 is an explanatory diagram illustrating the operation of the handpiece of Figure 1;

FIGURE 5 is a schematic view of a second embodiment of the invention, wherein a motor of a motor assembly drives a rotatable cannula, wherein the scope is disposed in the cannula, and wherein the cannula is shown in cross section;

FIGURE 6 is a view of the cannula and the motor assembly of Figure 5;

FIGURE 7 is a schematic view of a third embodiment of the invention wherein a lens closes off an annular sheath proximate a distal sheath end, wherein a scope is inserted into the sheath, wherein the sheath is inserted into a patient using a trocar, and wherein the sheath, the lens, and the trocar are shown in cross section;

FIGURE 8 is a view of the sheath and the lens of Figure 7;

FIGURE 9 is a cross sectional view of the sheath of Figure 8 taken along lines 9-9 of Figure 8;

FIGURE 10 a schematic view of a fourth embodiment of the invention, wherein a rotatably-driven transparent shield is rotatably attached to a sheath proximate a distal sheath end to seal the distal sheath end, wherein a scope is inserted into a proximal sheath end, and wherein the sheath and the shield are shown in cross section;

FIGURE 11 is a view of the sheath and the shield of Figure 10;

FIGURE 12 is a view of the shield of Figure 11 taken along lines 12-12 of Figure 11;

FIGURE 13 is a schematic view of a fifth embodiment of the invention wherein a slidable sleeve is attached to a scope, wherein the sleeve is shown slightly retracted from the distal scope end, and wherein the sleeve is shown in cross section;

FIGURE 14 is a view, as in Figure 13, but showing the sleeve extended from the distal scope end; and

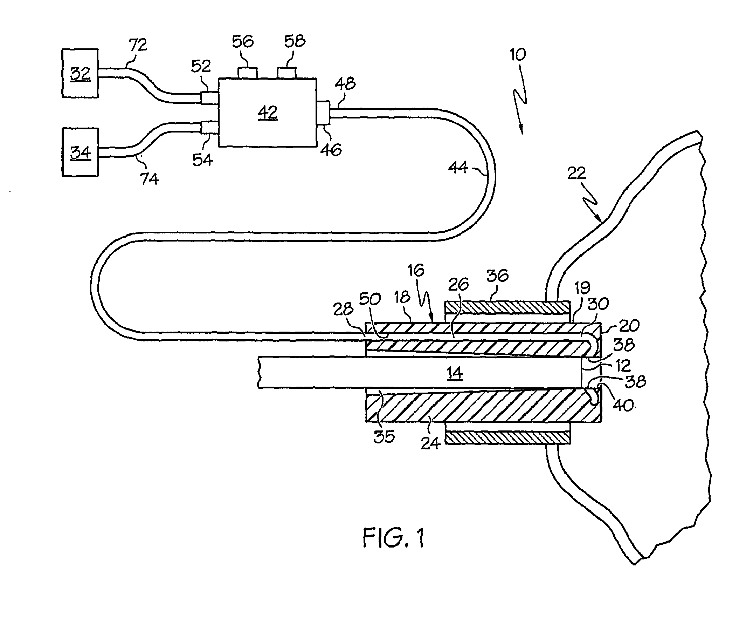

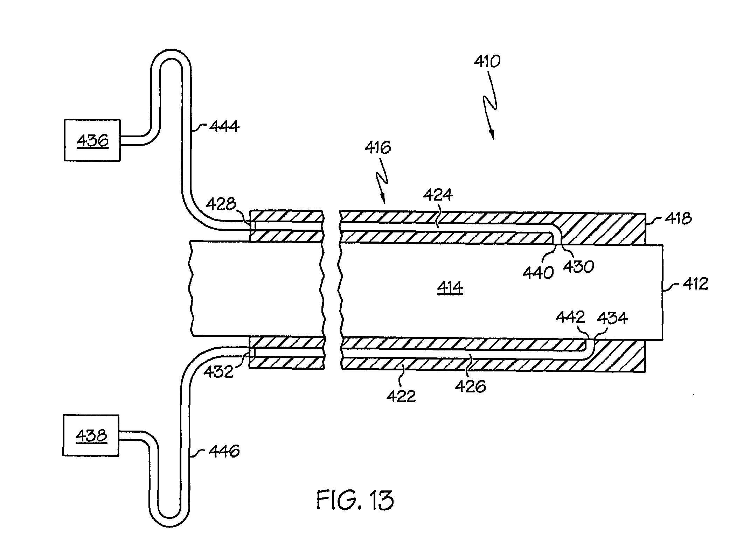

FIGURE 1 is a view of the sleeve of Figures 13-14.

### Detailed Description

Before explaining the several embodiments of the present invention in detail, it should be noted that each embodiment is not limited in its application or use to the details of construction and arrangement of parts and steps illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

It is further understood that any one or more of the following-described expressions, embodiments, examples, etc. can be combined with any one or more of the other following-described expressions, embodiments, examples, etc.

A first embodiment of the invention is shown in Figures 1-4. A first expression of the embodiment of Figures 1-4 is for apparatus 10 for cleaning a distal scope end 12 of a medical viewing scope 14. The apparatus 10 includes an annular sheath 16 having open proximal and distal sheath end portions 18 and 19. The sheath 16 is surroundingly attachable to the scope 14 and is insertable into a patient 22. The sheath 16 includes a tubular wall 24 having inside and outside diameters and containing a lumen 26 between the inside and outside diameters. The lumen 26 has proximal and distal lumen ends 28 and 30. The proximal lumen end 28 is fluidly connectable to at least one of an irrigation fluid source 32 and a vacuum source 34. The distal scope end 12 is in fluid communication with the distal lumen end 30 of the attached sheath 16. It is noted that the inside diameter of the tubular wall 24 is the diameter of a bore 35 into which the scope 14 is insertable. In an alternate first expression, a scope assembly includes the apparatus 10 and the scope 14, wherein the sheath 16 is attached to the scope 14. It is noted that "fluid" includes, without limitation, gas or gasses (e.g., air) and/or liquid or liquids.

It is noted that "cleaning a distal scope end" means cleaning at least a portion of the distal scope end to improve scope clarity. In the case of a scope having a lens at the distal scope end, "cleaning a distal scope end" means cleaning at least a portion of the lens to improve lens clarity.

In one enablement of the first expression of the embodiment of Figures 1-4, the scope 14 is slidingly insertable into the proximal sheath end portion 18, the distal scope end 12 has an outside diameter, and the inside diameter of the tubular wall 24 at the distal sheath end portion 19 is less than the outside diameter of the distal scope end 12. In one variation, the distal scope end 12 makes a press fit with the sheath 16 at the distal sheath end portion 19 as shown in Figure 1. In one modification, the inside diameter of the tubular wall 24 has a constant taper from the proximal sheath end 18 to proximate the distal sheath end 20 of the distal sheath end portion 19. Other attachments, not shown, of the sheath to the scope include, without limitation, an elastomeric sheath, a compression fitting, and an elastomeric O-ring attached to the sheath proximate the distal sheath end and adapted to attachingly engage an advancing scope which has been inserted into the proximal sheath end.

In one application of the first expression of the embodiment of Figures 1-4, the sheath 16 is insertable into a trocar 36. In one variation, the scope 14 is a laparoscope. Other types of scopes, not shown, include, without limitation, endoscopes (including gastroscopes and colonoscopes). It is noted that scopes include, without limitation, those scopes with video cameras which display an image on a monitor and those scopes having eyepieces for viewing by a physician.

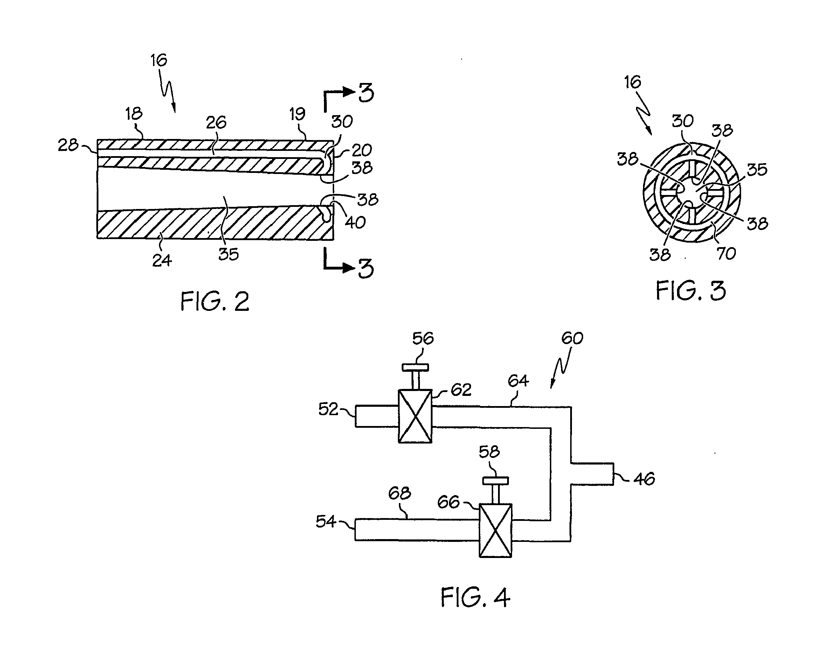

In one implementation of the first expression of the embodiment of Figures 1-4, the distal sheath end portion 19 has an orifice 38 (two are shown in Figures 1 and 2 and four are shown in figure 3) which is in fluid communication with the distal lumen end 30 and which points toward the distal scope end 12. In one variation, the distal sheath end portion 19 has a circumferentially inner surface 40 containing the orifice 38. It is noted that bodily fluid dispersion and floating debris should tend to adhere to the circumferentially inner surface 40. In one modification, the circumferentially inner surface 40 of the distal sheath end portion 19 longitudinally extends from the distal sheath end 20 of the distal sheath end portion 19 to the distal scope end 12 of the inserted scope 14, as seen in Figure 1.

In one arrangement of the first expression of the embodiment of Figures 1-4, the proximal lumen end 28 is fluidly connectable to the vacuum source 34. In one variation, the distal sheath end portion 19 has an orifice 38 which is in fluid communication with the distal lumen end 30. In one modification, the distal lumen end 30, for the attached sheath 16, is disposed within vacuuming distance of the distal scope end 12.

In one extension of the first expression of the embodiment of Figures 1-4, the apparatus 10 also includes a handpiece 42 and a flexible tube 44, wherein the handpiece 42 has a fluid outlet 46, and wherein the tube 44 has proximal and distal tube ends 48 and 50, as shown in Figure 1. The distal tube end 50 is attachable to the sheath 16 to be in fluid communication with the proximal lumen end 28, and the proximal tube end 48 is attachable to the handpiece 42 to be in fluid communication with the fluid outlet 46. In one variation, the handpiece 42 includes first and second fluid inlets 52 and 54, wherein the first fluid inlet 52 is fluidly connectable (such as via tubing 72) to the irrigation fluid source 32, and wherein the second fluid inlet 54 is fluidly connectable (such as via tubing 74) to the vacuum source 34. It is noted that the term "vacuum" includes partial vacuum and includes aspiration. It is also noted that the term "vacuum" is relative to the pressure at the orifice 38 (or at later-discussed orifice 238 or at later-discussed second orifice 442) and that, in one example, the vacuum source may be ambient room air when the orifice 38 (or the later-discussed orifice 238 or the later-discussed second orifice 442) is exposed to a higher pressure within, for example, the insufflated abdomen of a patient. In one modification, the handpiece 42 includes a first valve button 56 which, when pushed, fluidly connects the first fluid inlet 52 to the fluid outlet 46 and includes a second valve button 58 which, when pushed, fluidly connects the second fluid inlet 54 to the fluid outlet 46.

In one example, as shown in Figure 4, a "Y" passageway 60 fluidly connects the first and second fluid inlets 52 and 54 to the fluid outlet 46. The first valve button 56 is operatively connected to a first valve 62 disposed in a first arm 64 of the "Y" passageway 60. The second valve button 56 is operatively connected to a second valve 66 disposed in a second arm 68 of the "Y" passageway 60. Other examples are left to the artisan.

In one construction of the first expression of the embodiment of Figures 1-4, the distal sheath end portion 19 has a first coefficient of thermal conduction, the distal scope end 12 has a second coefficient of thermal conduction, and the first coefficient is higher than the second coefficient to reduce fogging of the distal scope end 12. It is noted that the term "fogging" includes condensation. In one example, the scope 14 has a light source (not shown) which warms the distal scope end 12, wherein the distal sheath end portion 19 acts as a heat sink for the distal scope end 12.

A second expression of the embodiment of Figures 1-4 is for apparatus 10 for cleaning a distal scope end 12 of a medical viewing scope 14. The apparatus 10 includes an annular sheath 16 having open proximal and distal sheath end portions 18 and 19. The sheath 16 is surroundingly attachable to the scope 14 and is insertable into a patient 22. The sheath 16 includes a tubular wall 24 having inside and outside diameters and containing a lumen 26 between the inside and outside diameters. The lumen 26 has proximal and distal lumen ends 28 and 30. The proximal lumen end 28 is fluidly connectable to at least one of an irrigation fluid source 32 and a vacuum source 34. The distal scope end 12 is in fluid communication with the distal lumen end 30 of the attached sheath 16. The distal sheath end portion 19 has an annular manifold 70 which is in fluid communication with the distal lumen end 30 and which includes at least one orifice 38 pointing toward the distal scope end 12. It is noted that the inside diameter of the tubular wall 24 is the diameter of a bore 35 into which the scope 14 is insertable. In an alternate second expression, a scope assembly includes the apparatus 10 and the scope 14, wherein the sheath 16 is attached to the scope 14.

In one variation of the second expression of the embodiment of Figures 1-4, the scope 14 is a laparoscope. In one arrangement of the second expression of the embodiment of Figures 1-4, the at-least-one orifice 38 includes a plurality of circumferentially spaced apart orifices 38. It is noted that the enablements, applications, etc. of the first expression of the embodiment of Figures 1-4 are applicable to the second expression of the embodiment of Figures 1-4.

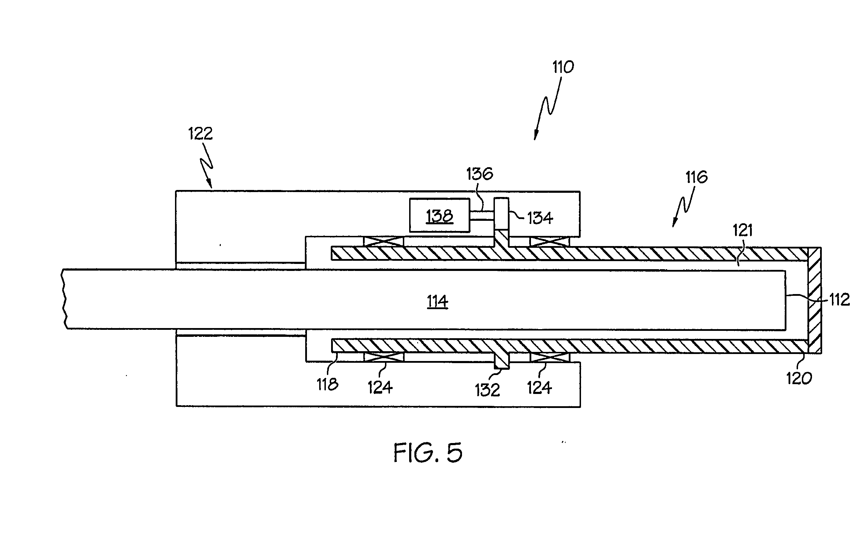

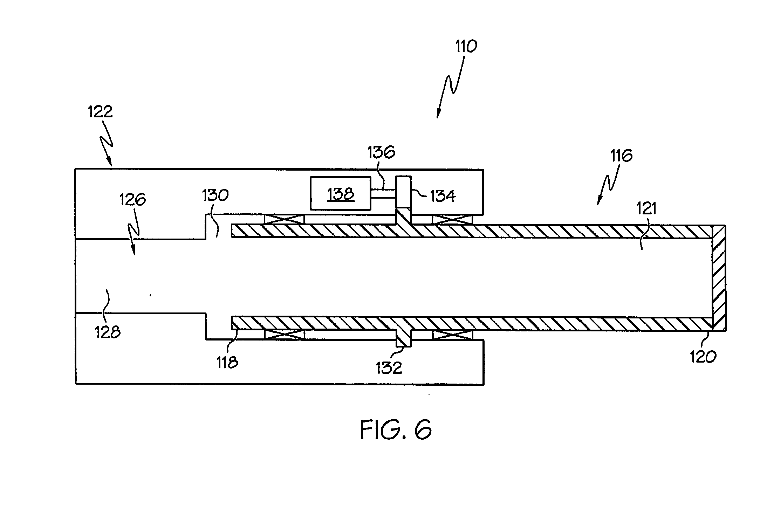

A second embodiment of the invention is shown in Figures 5-6. A first expression of the embodiment of Figures 5-6 is for apparatus 110 for cleaning a distal scope end 112 of a medical viewing scope 114. The apparatus 110 includes a motor-driven rotatable cannula 116 having an open proximal cannula end 118 and having a transparent and closed distal cannula end 120. The scope 114 is insertable into the proximal cannula end 118 with the distal scope end 112 disposed proximate and spaced apart from the distal cannula end 120. Rotation of the cannula 116 helps to expel any material that has accumulated on the distal cannula end 120 when the cannula 116 is inserted into a patient. It is noted that the cannula 116 surrounds a cannula bore 121 into which the scope 114 is insertable and that the closed distal cannula end 120 closes the cannula bore 121. In an alternate first expression, a scope assembly includes the apparatus 110 and the scope 114, wherein the scope 114 is inserted into the cannula 116.

In one enablement of the first expression of the embodiment of Figures 5-6, the apparatus 110 includes a motor assembly housing 122 and bearings 124. The motor assembly housing 122 includes a housing bore 126 having proximal and distal housing bore portions 128 and 130. The bearings 124 rotatably attach the cannula 116 to the motor assembly housing 122 in the distal housing bore portion 130. The scope 114 is inserted first into the proximal housing bore portion 128 and then into the proximal cannula end 118. The cannula 116 has a circumferential gear 132 which is rotated by a motor gear 134 attached to the drive shaft 136 of an electric motor 138 disposed in the motor assembly housing 122. Although not shown in the figures, in this enablement wires would extend from the electric motor 138 to a switch on a handpiece containing a battery. Other enablements for having a motor drive the rotatable cannula 116 include, for example and without limitation, having an AC or DC or non-electric motor disposed in a trocar or on a cart and are left to the artisan. In one variation of the first expression of the embodiment of Figures 5-6, the scope 114 is a laparoscope.

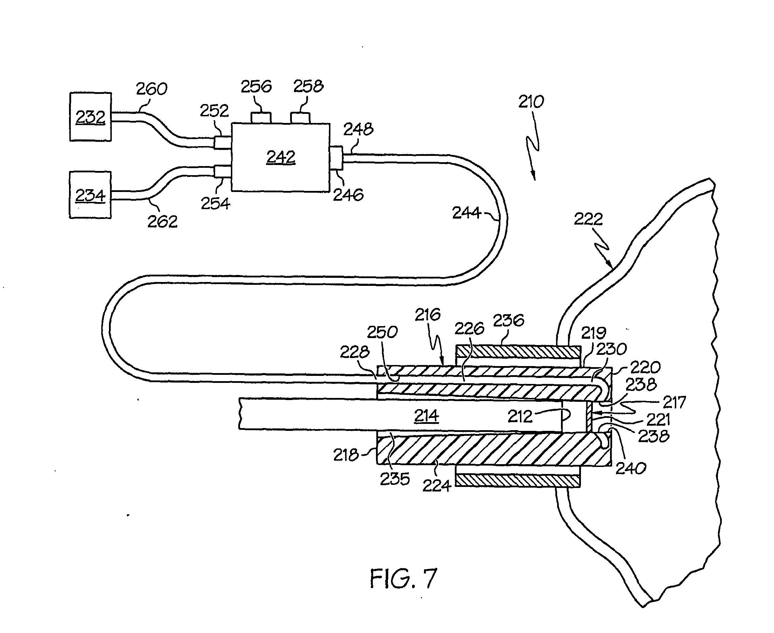

A third embodiment of the invention is shown in Figures 7-9. A first expression of the embodiment of Figures 7-9 is for apparatus 210 for cleaning a distal scope end 212 of a medical viewing scope 214. The apparatus 210 includes an annular sheath 216 and a lens 217. The sheath 216 has open proximal and distal sheath ends 218 and 220. The lens 217 is disposed in and attached to the sheath 216 and closes off the sheath 216 proximate the distal sheath end 220. The lens 217 has a distal surface 221. The scope 214 is insertable into the proximal sheath end 218 with the distal scope end 212 disposed proximate the lens 217. The sheath 216 is insertable into a patient 222. The sheath 216 includes a tubular wall 224 having inside and outside diameters and containing a lumen 226 between the inside and outside diameters. The lumen 226 has proximal and distal lumen ends 228 and 230. The proximal lumen end 228 is fluidly connectable to at least one of an irrigation fluid source 232 and a vacuum source 234. The distal surface 221 of the lens 217 is in fluid communication with the distal lumen end 230. It is noted that the lens 217 may be a non-magnifying or a magnifying lens. It is noted that the sheath 216 surrounds a bore 235 into which the scope 214 is insertable and that the lens 217 closes off the sheath 216 by closing off the bore 235. In an alternate first expression, a scope assembly includes the apparatus 210 and the scope 214, wherein the scope 214 is inserted into the sheath 216.

In one application of the first expression of the embodiment of Figures 7-9, the sheath 216 is insertable into a trocar 236. In one variation, the scope 214 is a laparoscope.

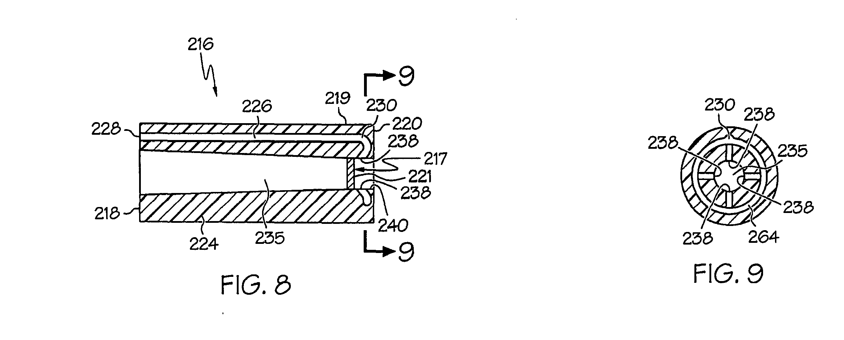

In one implementation of the first expression of the embodiment of Figures 7-9, the distal sheath end portion 219 has an orifice 238 (two are shown in Figures 7 and 8 and four are shown in figure 9) which is in fluid communication with the distal lumen end 230 and which points toward the distal scope end 212. In one variation, the distal sheath end portion 219 has a circumferentially inner surface 240 containing the orifice 238. It is noted that bodily fluid dispersion and floating debris should tend to adhere to the circumferentially inner surface 240. In one modification, the circumferentially inner surface 240 of the distal sheath end portion 219 longitudinally extends from the distal sheath end 220 of the distal sheath end portion 219 to the distal surface 221 of the lens 217, as seen in Figures 7-8.

In one extension of the first expression of the embodiment of Figures 7-9, the apparatus 210 also includes a handpiece 242 and a flexible tube 244, wherein the handpiece 242 has a fluid outlet 246 and the tube 244 has proximal and distal tube ends 248 and 250, as shown in Figure 7. The distal tube end 250 is attachable to the sheath 216 to be in fluid communication with the proximal lumen end 228, and the proximal tube end 248 is attachable to the handpiece 242 to be in fluid communication with the fluid outlet 246. In one variation, the handpiece 242 includes first and second fluid inlets 252 and 254, wherein the first fluid inlet 252 is fluidly connectable (via tubing 260) to the irrigation fluid source 232, and wherein the second fluid inlet 254 is fluidly connectable (via tubing 262) to the vacuum source 234. In one modification, the handpiece 242 includes a first valve button 256 which, when pushed, fluidly connects the first fluid inlet 252 to the fluid outlet 246 and includes a second valve button 258 which, when pushed, fluidly connects the second fluid inlet 254 to the fluid outlet 246. In one example, the distal sheath end portion 219 includes a manifold 264 fluidly connecting the distal lumen end 230 to a plurality of orifices 238 on the circumferential inner surface 240 of the distal sheath end 220, wherein the orifices 238 each point toward the distal scope end 212.

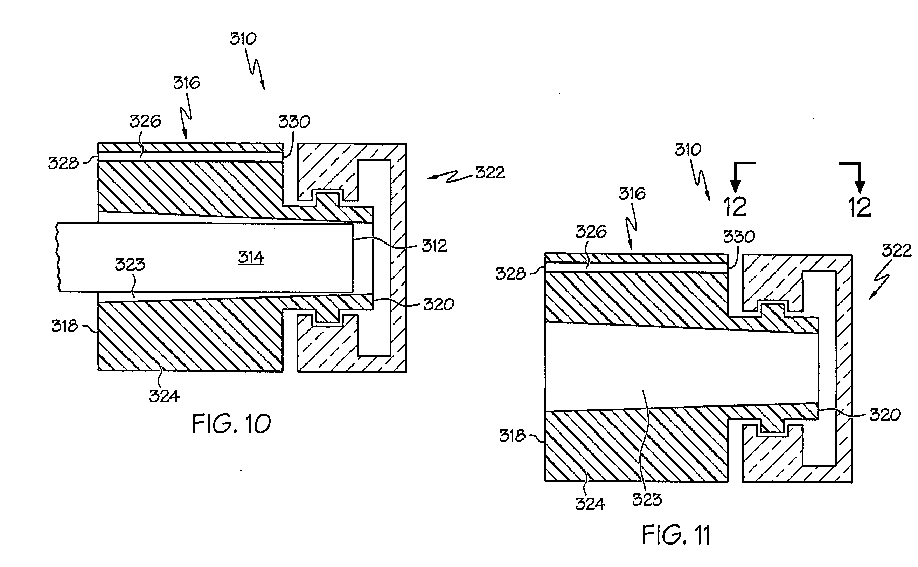

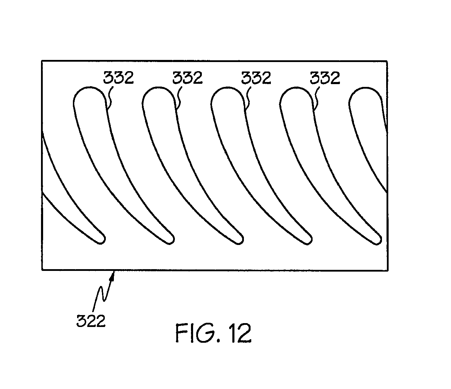

A fourth embodiment of the invention is shown in Figures 10-12. A first expression of the embodiment of Figures 10-12 is for apparatus 310 for cleaning a distal scope end 312 of a medical viewing scope 314. The apparatus 310 includes an annular sheath 316 having open proximal and distal sheath ends 318 and 320 and a rotatably-driven transparent shield 322 rotatably attachable to the sheath 316 proximate the distal sheath end 320 to seal the distal sheath end 320. The scope 314 is insertable into the proximal sheath end 318 with the distal scope end 312 disposed proximate and spaced apart from the shield 322. Rotation of the shield 322 helps to expel any material that has accumulated on the shield 322 when the sheath 316 and the attached shield 322 are inserted into a patient. It is noted that the sheath 316 surrounds a bore 323 into which the scope 314 is insertable and that the rotatable shield 322 seals the distal sheath end 320 by providing a rotatable sealing cover for the bore 323 proximate the distal sheath end 320. In one example, the shield 322 is rotatably attached to the sheath 316. In an alternate first expression, a scope assembly includes the apparatus 310 and the scope 314, wherein the scope 314 is inserted into the sheath 316.

In a first enablement of the first expression of the embodiment of Figures 10-12, the sheath 316 includes a tubular wall 324 having inside and outside diameters and containing a lumen 326 between the inside and outside diameters. The lumen 326 has proximal and distal lumen ends 328 and 330. The proximal lumen end 328 is fluidly connectable to a source of pressurized gas (not shown). The distal lumen end 330 points toward an aligned one of a plurality of circumferentially arrayed airfoil blades 322 which are attached to, or are a monolithic portion of, the shield 322, wherein the impact of the pressurized air on the aligned blade rotates the blade bringing an adjacent blade into alignment resulting in continuous rotation of the shield 322 as long as the pressurized gas is allowed to flow in the lumen 326. In one variation, the gas is air. Other choices for the gas are left to the artisan. In a second enablement, the proximal lumen end 328 is fluidly connectable to a source of pressurized liquid. Other enablements, not shown, employ electric motors or modify the sheath and the shield to be electric motor components to rotatably drive the shield, as is within the level of design skill of the artisan.

In one variation of the first expression of the embodiment of Figures 10-12, the scope 314 is a laparoscope.

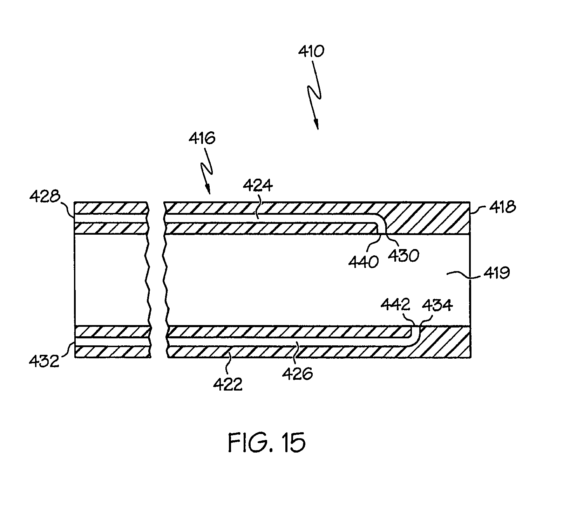

A fifth embodiment of the invention is shown in Figures 13-15. A first expression of the embodiment of Figures 13-15 is for apparatus 410 for cleaning a distal scope end 412 of a medical viewing scope 414. The apparatus 410 includes a sleeve 416 attachable to and slidable along the scope 414. The sleeve 416 includes a distal sleeve tip 418. The distal sleeve tip 418 of the attached sleeve 416 is slidably extendable from and slidably retractable from the distal scope end 412. In an alternate first expression, a scope assembly includes the apparatus 410 and the scope 414, wherein the sleeve 416 is attached to the scope 14.

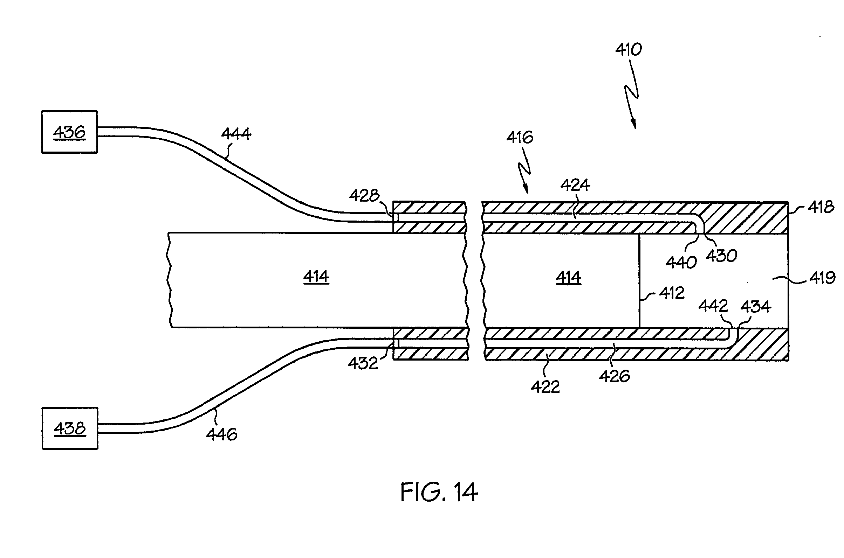

In a first enablement of the first expression of the embodiment of Figures 13-15, the sleeve 416 is a fully-annular cylindrical sleeve which, it is noted, surrounds a bore 419 into which the scope 414 is insertable. In a second enablement, the sleeve is a partially-annular cylindrical sleeve. In one employment of the first expression of the embodiment of Figures 13-15, a physician holds a proximal portion of the scope 414 in one hand and a proximal portion of the sleeve 416 in the other hand to slide the sleeve 416 relative to the scope 414 (or equivalently to slide the scope relative to the sleeve) to retract the sleeve 416 from the distal scope end 412 (as shown in Figure 13) and to extend the sleeve 416 from the distal scope end 412 (as shown in Figure 14).

In one employment of the first expression of the embodiment of Figures 13-15, the distal sleeve tip 418 of the extended sleeve 416 should clear blockages (such as clots) from the distal scope end 412, and the distal sleeve tip 418 of the retracted sleeve 416 should be relatively free of fluid drops.

In a first implementation of the first expression of the embodiment of Figures 13-15, the sleeve 416 includes a tubular wall 422 having inside and outside diameters and containing first and second lumens 424 and 426 between the inside and outside diameters, wherein the first lumen 424 has first proximal and distal lumen ends 428 and 430, wherein the second lumen 426 has second proximal and distal lumen ends 432 and 434, wherein the first proximal lumen end 428 is fluidly connectable (such as via tubing 444) to an irrigation fluid source 436, wherein the second proximal lumen end 432 is fluidly connectable (such as via tubing 446) to a vacuum source 438, wherein the first distal lumen end 430 includes a first orifice 440 disposed at the inside diameter of the tubular wall 422, and wherein the second distal lumen end 434 includes a second orifice 442 disposed at the inside diameter of the tubular wall 422 distal the first orifice 440, wherein the sleeve 416 is hermetically slidable along the scope 414, and wherein the sleeve 416, when extending beyond the distal scope end 412 first exposes the second orifice 442 and then the first orifice 440. In this implementation, the separate first and second lumens 424 and 426 ensure that the irrigation fluid does not contain recently suctioned material, as can be appreciated by those skilled in the art.

In a second implementation, not shown, the vacuum orifice is replaced with a pressurized gas orifice. In one variation, the irrigation fluid and/or the pressurized gas is heated to reduce the potential for fogging. In the same or a different variation, an anti-fogging agent (such as a surfactant) is added to the irrigation fluid.

In one variation of the first expression of the embodiment of Figures 13-15, the scope 414 is a laparoscope.

Several benefits and advantages are obtained from one or more of the expressions of embodiments of the invention which provide cleaning of a distal scope end of a medical viewing scope while the scope remains inserted in a patient. In one example, not removing the scope for cleaning and not reinserting the cleaned scope reduces the time for a laparoscopic procedure. In the same or a different example, not removing the scope for cleaning and not reinserting the cleaned scope keeps the inserted scope aligned with the patient tissue of interest during cleaning so that the physician does not have to take additional time to maneuver the scope to reacquire the patient tissue of interest.

While the present invention has been illustrated by a description of several expressions, embodiments, and examples, etc. thereof, it is not the intention of the applicants to restrict or limit the spirit and scope of the appended claims to such detail. Numerous other variations, changes, and substitutions will occur to those skilled in the art without departing from the scope of the invention. It will be understood that the foregoing description is provided by way of example, and that other modifications may occur to those skilled in the art without departing from the scope and spirit of the appended Claims.

## Claims

1. Apparatus for cleaning a distal scope end of a medical viewing scope, wherein the apparatus comprises an annular sheath having open proximal and distal sheath end portions, wherein the sheath is surroundingly attachable to the scope and is insertable into a patient, wherein the sheath includes a tubular wall having inside and outside diameters and containing a lumen between the inside and outside diameters, wherein the lumen has proximal and distal lumen ends, wherein the proximal lumen end is fluidly connectable to at least one of an irrigation fluid source and a vacuum source, and wherein the distal scope end is in fluid communication with the distal lumen end of the attached sheath.

2. The apparatus of claim 1, wherein the scope is slidingly insertable into the proximal sheath end portion, wherein the distal scope end has an outside diameter, and wherein the inside diameter of the tubular wall at the distal sheath end portion is less than the outside diameter of the distal scope end.

3. The apparatus of claim 1, wherein the sheath is insertable into a trocar.

4. The apparatus of claim 3, wherein the scope is a laparoscope.

5. The apparatus of claim 1, wherein the distal sheath end portion has an orifice which is in fluid communication with the distal lumen end and which points toward the distal scope end.

6. The apparatus of claim 5, wherein the distal sheath end portion has a circumferentially inner surface containing the orifice.

7. The apparatus of claim 1, wherein the proximal lumen end is fluidly connectable to the vacuum source, and wherein the distal sheath end portion has an orifice which is in fluid communication with the distal lumen end and which, for the attached sheath, is disposed within vacuuming distance of the distal scope end.

8. The apparatus of claim 1, also including a handpiece and a flexible tube, wherein the handpiece has a fluid outlet, wherein the tube has proximal and distal tube ends, wherein the distal tube end is attachable to the sheath to be in fluid communication with the proximal lumen end, wherein the proximal tube end is attachable to the handpiece to be in fluid communication with the fluid outlet.

9. The apparatus of claim 8, wherein the handpiece includes first and second fluid inlets, wherein the first fluid inlet is fluidly connectable to the irrigation fluid source, and wherein the second fluid inlet is fluidly connectable to the vacuum source.

10. The apparatus of claim 9, wherein the handpiece includes a first valve button which, when pushed, fluidly connects the first fluid inlet to the fluid outlet and includes a second valve button which, when pushed, fluidly connects the second fluid inlet to the fluid outlet.

11. Apparatus for cleaning a distal scope end of a medical viewing scope, wherein the apparatus comprises an annular sheath having open proximal and distal sheath end portions, wherein the sheath is surroundingly attachable to the scope and is insertable into a patient, wherein the sheath includes a tubular wall having inside and outside diameters and containing a lumen between the inside and outside diameters, wherein the lumen has proximal and distal lumen ends, wherein the proximal lumen end is fluidly connectable to at least one of an irrigation fluid source and a vacuum source, wherein the distal scope end is in fluid communication with the distal lumen end of the attached sheath, wherein the distal sheath end portion has an annular manifold which is in fluid communication with the distal lumen end and which includes at least one orifice pointing toward the distal scope end.

12. Apparatus for cleaning a distal scope end of a medical viewing scope, wherein the apparatus comprises a motor-driven rotatable cannula having an open proximal cannula end and having a transparent and closed distal cannula end, wherein the scope is insertable into the proximal cannula end with the distal scope end disposed proximate and spaced apart from the distal cannula end, and wherein rotation of the cannula helps to expel any material that has accumulated on the distal cannula end when the cannula is inserted into a patient.

13. Apparatus for cleaning a distal scope end of a medical viewing scope, wherein the apparatus comprises an annular sheath and a lens, wherein the sheath has open proximal and distal sheath ends, wherein the lens is disposed in and attached to the sheath and closes off the sheath proximate the distal sheath end, wherein the lens has a distal surface, wherein the scope is insertable into the proximal sheath end with the distal scope end disposed proximate the lens, wherein the sheath is insertable into a patient, wherein the sheath includes a tubular wall having inside and outside diameters and containing a lumen between the inside and outside diameters, wherein the lumen has proximal and distal lumen ends, wherein the proximal lumen end is fluidly connectable to at least one of an irrigation fluid source and a vacuum source, and wherein the distal surface of the lens is in fluid communication with the distal lumen end.

14. Apparatus for cleaning a distal scope end of a medical viewing scope, wherein the apparatus comprises an annular sheath having open proximal and distal sheath ends and a rotatably-driven transparent shield rotatably attachable to the sheath proximate the distal sheath end to seal the distal sheath end, wherein the scope is insertable into the proximal sheath end with the distal scope end disposed proximate and spaced apart from the shield, and wherein rotation of the shield helps to expel any material that has accumulated on the shield when the sheath and the attached shield are inserted into a patient.

15. Apparatus for cleaning a distal scope end of a medical viewing scope, wherein the apparatus comprises a sleeve attachable to and slidable along the scope, wherein the sleeve includes a distal sleeve tip, and wherein the distal sleeve tip of the attached sleeve is slidably extendable from and slidably retractable from the distal scope end.
